# EUROPÄISCHE PATENTSCHRIFT

(11) **EP 2 636 443 B1**
(45) Veröffentlichungstag und Bekanntmachung des Hinweises auf die Patenterteilung: **18.06.2014**
(21) Anmeldenummer: 13000775.0
(22) Anmeldetag: 14.02.2013
(51) Int. Cl.: B01F 1/00, B01F 5/10, B01F 5/04, B01F 15/00, A61M 1/16

(54) **Mischvorrichtung zur Herstellung gebrauchsfertiger medizinischer Spüllösungen insbesondere für die Hämodialysekonzentrate**
Mixing device for producing ready-to-use medical irrigation solutions in particular for haemodialysis concentrates
Dispositif de mélange destiné à fabriquer des solutions de rinçage médicales prêtes à l'emploi, en particulier pour les concentrés d'hémodialyse

(30) Priorität: 10.03.2012 DE 102012004886
(43) Veröffentlichungstag der Anmeldung: 11.09.2013
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- EP-A1- 0 469 487
- EP-A1- 0 659 956
- WO-A1-00/74833
- DE-A1- 3 844 174

## Beschreibung

Gegenstand der Erfindung ist eine technologisch einfache Mischvorrichtung für die Herstellung gebrauchsfertiger medizinischer Spüllösungen, insbesondere von Konzentraten für die Hämodialyse.

Nachteile des Standes der Technik sind:
Die Herstellung ist zeitaufwändig, hoher Energieeinsatz, die Auflösung des Rohstoffes dauert sehr lang.
Die Einhaltung von normativen Vorgaben für fertige Lösungen wird wegen ungenauer Verdünnung bzw. Flüssigkeitszudosierung erschwert.
Es sind deshalb aufwändige hausinterne Kontrollen hinsichtlich der Genauigkeit erforderlich.
Mischanlage und Flüssigkeit werden aufgrund unzureichender mikrobiologischer Prävention kontaminiert, es sind aufwändige Hygienemaßnahmen erforderlich.

Die Fertiglösungsbehälter haben keine visuelle Zugänglichkeit hinsichtlich des erforderlichen Niveaus, keine Kontrolle hinsichtlich der Tankausrichtung, keine Langzeitstabilität bei Kunststofftanks.

Es können nicht alle Varianten des Rohstoffes z.B. pulvrig, granuliert, geschlämmt (pastös), verarbeitet werden.

Eine Wiederbefüllung des Rohstoffbehälters vor Ort ist nicht möglich.

Ein Transfer der Fertiglösung zum Anwendungsort ist nicht vorgesehen.

Dokument WO-A-00/74833 offenbart eine Mischvorrichtung gemäß dem Oberbegriff des Anspruchs 1. Mit der im Anspruch 1 definierten Erfindung werden folgende Verbesserungen angestrebt:
Das Mischverfahren soll
   Kurzzeitig, energiesparend sein;
   normativen Vorgaben ohne hausinterne Kontrolle sollen für die fertige Lösungen eingehalten werden;
   Hygienisch einwandfrei,
   technisch einfach sein

Dies wird erreicht durch eine ausfallsichere hochgenaue Zudosierung der Flüssigkeit.

Durch gute Hygiene-/Sterilität wie z. B.: durch sehr gute Spül- und ggf. Desinfektionsmöglichkeiten.

Die eingesetzten Komponenten z. B.: Fertiglösungsbehälter können mit einer visuellen Lage und Niveauerkennung und einer kalibrierbare Niveauerkennung mittels Sensoren ausgestattet werden.

Der Rohstoffbehälter kann wiederbefüllbar ausgestattet und mit pulvrigem, granuliertem, oder geschlämmtem (pastösem) Rohstoff vor Ort befüllt werden.

Die fertige Lösung kann mittels eines einfachen Transfers zum Ort der Anwendung befördert werden.

Die Verbesserungen sind wie folgt realisiert:
Das Mischgerät enthält einen Rechner mit Ein- und Ausgabeeinheiten, eine Hauptmischleitung bzw. Rezirkulationskreislauf mit einer Zirkulationspumpe, einer Venturipumpe (Venturirohr) und wenigstens einem Ventil und eine Sekundärmischleitung, die mit einem Anschluss von der Saugleitung des Venturirohres abzweigt und mit dem gefüllten Rohstoffbehälter verbunden ist und mit dem anderen Anschluss vom Rohstoffbehälter über ein weiteres Ventil stromaufwärts wieder in die Hauptmischleitung einmündet.

Die zur Verdünnung bzw. zur Aufbereitung benötigte Flüssigkeit wird aus einer Flüssigkeitsquelle, die vorzugsweise in ihrer zentralen Funktion aus einer Umkehrosmose besteht, mittels einer nachfolgend beschriebenen präzisen Zudosiereinrichtung einem Ansatztank bzw. Fertiglösungsbehälter zugeführt.

Dabei wird zu Beginn des Mischvorganges zunächst die Hauptmischleitung mittels Hauptmischventil gesperrt und die Flüssigkeit in Strömungsrichtung der Hauptmischleitung über die Sekundärmischleitung gefördert.

Mit Vorteil besteht die Möglichkeit, Hauptmischung und Sekundärmischung gleichzeitig so zu betreiben, dass die Sekundärmischung im Gegenstrom zur Hauptmischung betrieben wird. Dazu wird ein weiterer Strömungswiderstand stromaufwärts in die Hauptmischleitung so eingesetzt, so dass die Flüssigkeit dem Rohstoffbehälter von oben zugeführt und unten mittels Venturipumpe abgesaugt wird.

Im Wechsel von Gegen- und Gleichstrommischung durch den Rohstoffbehälter bei gleichzeitigem Betrieb der Hauptmischung - wenn die Flüssigkeit im Gegenstrom durch den Rohstoffbehälter fließt- kann eine schnelle, kostengünstige, effiziente Aufbereitung von Konzentrat für die Hämodialyse oder eine medizinische Spüllösungen für andere Applikationen hergestellt werden.

Stromabwärts des Rohstoffbehälters ist eine Spülleitung angeschlossen, die bei konnektiertem Rohstoffbehälter nicht durchflossen werden kann. Die Spülleitung wird mit Vorteil an ihrem Ende mit einem Sprühkopf abgeschlossen und mündet dabei so in den Fertiglösungsbehälters ein, dass die Innenoberfläche des Fertiglösungsbehälters nahezu vollständig gereinigt und bei Bedarf desinfiziert werden kann. Durch die Spülleitung kann der Rohstoffbehälter belüftet werden.

Zur Verbesserung der Hygiene und Wiederherstellung der Reinheit nach einem Mischvorgang, oder nach einer längeren betriebsfreien Zeit kann stromabwärts der Spülleitung nach der Kupplungsstelle eine physikalische oder chemische Desinfektionseinrichtung vorgesehen werden.

Dadurch ist ein Eintrag von gefährlichen Desinfiziens während des Mischvorganges nicht möglich.

Um eine rückstandsfreie Entleerung des Fertiglösungsbehälters zu gewährleisten, kann an die Mischanlage an einen freien Auslauf angeschlossen werden.

Eine wesentliche Komponente der Mischvorrichtung ist die ausfallüberwachte Zudosierungseinrichtung. Diese Einrichtung überwacht das Volumen der zugeführten, zur Aufbereitung des Rohstoffes benötigten Flüssigkeit, ist mitentscheidend für die Genauigkeit - d.h. die Gebrauchstauglichkeit der Lösung - und macht bei Einsatz validierter Rohstoffe eine weitere zeit- und kostenintensive Überprüfung der fertigen Lösung überflüssig.

Die Zudosierungseinrichtung besteht in der Basisausführung aus einem Wassereingangsventil, einem Abflussventil, mindestens zwei Niveausensoren zur Bestimmung des zugeführten Volumens, wobei der stromaufwärts eingebaute Niveausensor gleichzeitig die Dichtigkeit des Wassereingangsventils überwacht.

Mit Vorteil erhält der in der Regel aus Kunststoff hergestellte Fertiglösungsbehälter mindestens eine umlaufende Markierung, durch die sowohl das oder die erforderlichen Flüssigkeitsniveau(s) als auch eine Schräglage des Tanks durch den Anwender überwachbar sind.

Innerhalb dieser Markierung befindet sich der zweite zur Bestimmung des Füllstandes bzw. des Volumens erforderliche Niveausensor. Beispielsweise sind dies optische, kapazitive, o.a. Messsensoren, die geeignet sind, Flüssigkeit zu detektieren.

Darüber hinaus kann die Sensorik für weitere Funktionen wie z.B. auch zur Tankleererkennung eingesetzt werden.

Da insbesondere bei Kunststofftanks durch Montage-, Fertigungs-, Belastungs-, u. Alterungsschwankungen Volumenänderungen nicht auszuschließen sind, sind die im Tank befindlichen Sensoren in ihrer Lage einstellbar.

Darüber hinaus kann zur Verhinderung der häufig auftretenden Tankausbauchung tankmittig ein Spanngurt angesetzt werden, der gegen Verrutschen durch am Tank befindliche Noppen gesichert ist.

Eine andere Art einer Zudosierungseinrichtung, die sowohl tankunabhängig als auch für variable Zuflussmengen eingesetzt werden kann, besteht in der Basisausführung aus einer Messkammer mit beispielsweise mindestens einem optischen, kapazitiven, o.a. Messsensor, der geeignet ist, Flüssigkeit zu detektieren, und mindestens einem angeschlossenen Flussmesser und mindestens einem Ventil.

Durch Einsatz einer Messkammer können mit Vorteil unterschiedliche Fertiglösungsbehälter in Volumen und Ausführung wie z.B. mobile- oder stationäre-, als starre Tanks oder flexible Beutel, Ausführung in den Mischkreis eingebracht werden, weil das Anbringen von Messeinrichtungen an diesen Behältern entfallen kann.

Aufwändigere auch für variable Füllvolumen geeignete, mit dem Fertiglösungsbehälter/ Tank kombinierte Zudosierungseinrichtungen, sind z.B. Wäagetechnik, kontinuierliche stetige Füllstandsmessungen wie z.B. Druckmesstechnik, Echolot, oder volumetrische Zudosierungen wie z.B. Bilanzkammern u.a. hier nicht aufgeführte Verfahren.

Alternativ könnten je nach Kosten-, bzw. Sicherheitsaspekten o.a. aufgeführte Ausführungen einer Zudosierungseinrichtung zum Einsatz kommen.

Mit Vorteil wird zur Verbesserung der Hygiene und Sterilität der zugeführten Flüssigkeit eine Kombination einer RO (Umkehrosmose) mit der Mischanlage vorgesehen. Darüber hinaus kann die Reinheit der von der RO erzeugten Flüssigkeit über weitere zusätzliche Filterstufen wie z.B. Entionisierung und/oder eine Sterilfiltration hinsichtlich Leitfähigkeit und Mikrobiologie so verbessert werden, dass eine gleich gute oder sogar bessere Wasserqualität, wie sie für die Produktion von Arzneimitteln vorgeschrieben ist, erzielt werden kann.

Falls eine temperierte Flüssigkeit zur Anwendung kommen soll, oder um die Auflösungszeiten zu reduzieren, kann eine optional zur Verfügung stehende Heizung eingesetzt werden. Ebenfalls kann zur Unterstützung der Auflösung des Rohstoffes ein statischer Mischer zur Anwendung kommen.

Die Mischqualität ist mikrobiell rückstandsfrei, die Vorrichtung arbeitet energiesparend. Da sowohl der Rohstoff validiert - also ein medizinisches Produkt - ist, als auch die zugeführte Flüssigkeit hinsichtlich Reinheit und Exaktheit des Volumens den Genauigkeitsanforderungen entsprechen, entfallen aufwändige hausinterne Kontrollen.

Es ist möglich, durch Hinzunahme weiterer Filterstufen hochsterile Medikamente herzustellen.

Nachfolgend werden Ausführungsformen der Erfindung mit Bezug auf die Zeichnungen näher beschrieben. Diese zeigen.
- Fig.1: Vorrichtung zum Mischen eines Rohstoffes in einem Wechselbehälter und einem Fertiglösungsbehälter
- Fig.2: Vorrichtung zum Mischen eines Rohstoffes in einem vor Ort befüllbaren Wechselbehälter und einem Fertiglösungsbehälter mit Niveaumarkierungen und einer Desinfektions-/ Säurevorrichtung
- Fig.3: Vorrichtung zum Mischen eines Rohstoffes in einem Rohstoffbehälter -Beutel ohne Fertiglösungsbehälter.
- Fig.4: Vorrichtung zum Mischen eines Rohstoffes in einem Beutel, einem mobilen Fertiglösungsbehälter, steriler Flüssigkeitszuführung und zusätzlicher Sterilfiltration.

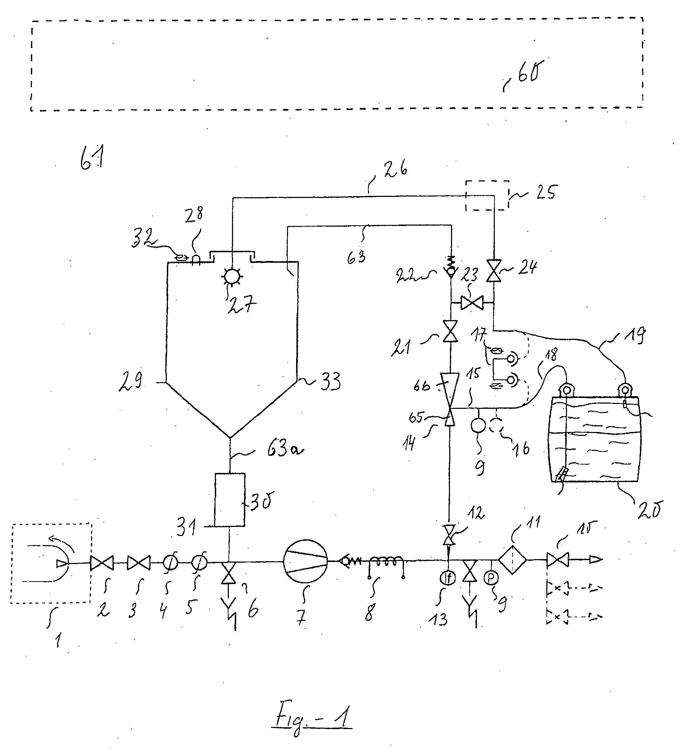

Dabei zeigt Figur 1 die Flüssigkeitszuführung (1) und die ausfallsichere Zudosierung, die aus den Komponenten Betriebsventil (2), Schutzventil (3), Betriebsflussmesser (4), Schutzsystemflussmesser (5) und den Niveau Sensoren (31/29) besteht. Vorteilhafterweise werden die Ventile (2/3) in unterschiedlichen Ausführungen, z. B. Membranventil und/oder einem Servoventil eingesetzt, um einen Ausfall bzw. eine ungewollte Zudosierung zu verhindern. Die beiden Flussmesser (4/5) werden durch den Rechner (60) der Mischanlage (61) überprüft und durch das als bekannt vorausgesetzte Volumen zwischen den Niveau Sensoren (29/31) verifiziert.

Die optionale Messkammer (30) mit bekanntem Volumen kann dabei tankunabhängig die Flussmesser (4/5) verifizieren. Bei Einsatz einer Messkammer (30) wird auch der Niveausensor (29) an dieser angebracht.

Der Fertiglösungsbehälter (Ansatzbehälter) (33) wird über die Füllleitung (63) bzw. bei Verwendung einer Messkammer (30) über diese gefüllt, bis das erforderliche Volumen erreicht ist. Dabei befindet sich der Niveausensor (31) vorzugsweise in einem vertikalen Leitungsabschnitt zwischen Tankauslauf und Ablaufventil (6). Die Entlüftung des Fertiglösungsbehälters (33) erfolgt über den hydrophylen Entlüftungsfilter (28). Ein Überlauf kann mit Leckagemelder (32) detektiert werden.

Eine Entleerungsmöglichkeit des Fertiglösungsbehälters (33) besteht über das Abflussventil (6), welches aus Gründen der Rückkontamination stromabwärts in einen freien Auslauf münden sollte. Dieser Weg wird vorzugsweise nach einem Spülvorgang oder als Verwurfsmöglichkeit bei fehlerhafter Fertiglösung gewählt. Vorteilhafterweise wird zu Beginn des Füllprozesses zunächst ein Teil der zugeführten Flüssigkeit über Abflussventil (6) zur Verhinderung eines Keimeintrages verworfen.

Der Zufluss zum Fertiglösungsbehälter (33) kann ebenfalls über die Flussmesser von oben in den Behälter erfolgen. Die Lage der Niveausensoren (29/31) verbleibt trotzdem stromabwärts des Tankauslaufes/Füllleitung (63).

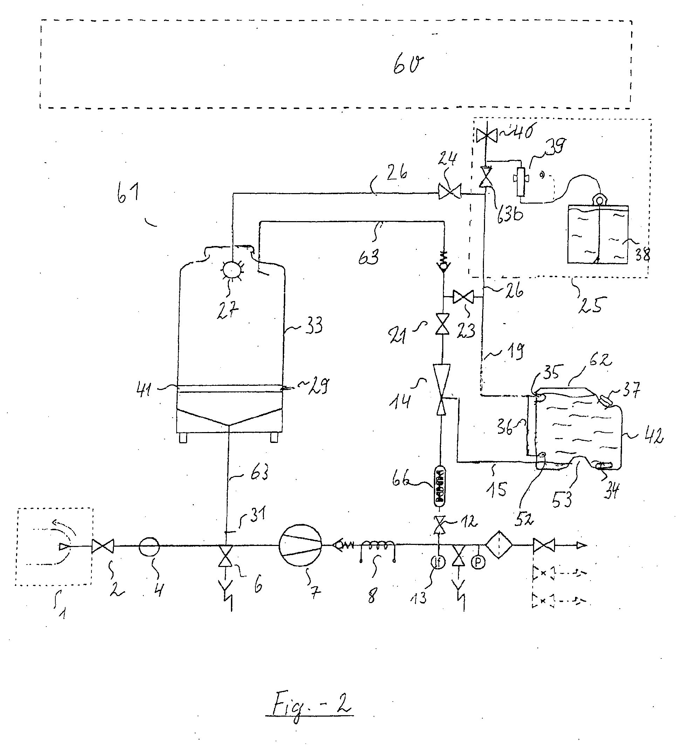

Zu Beginn des Mischprozesses fördert die Pumpe (7) einen Teil der zuvor zudosierten Flüssigkeit aus dem Fertiglösungsbehälter (33) über die optionale Heizung (8) und den optionalen statischen Mischer (66) (Figur 2), die optionale Leitfähigkeits- Temperaturmessung (13), das Füllhilfsventil (12), das Venturi (14) in den Rohstoffbehälter (20); dabei bleiben das Mischventil (21) und das Spülventil (24) geschlossen. Über das geöffnete Sekundärmischventil (23) und die Mischleitung (63) zirkuliert das Flüssigkeitsrohstoffgemisch in den Fertiglösungsbehälter (33). Die Fließrichtung in den Rohstoffbehälter (20) kann zur besseren Auflösung des Rohstoffes durch zusätzliches Öffnen von Mischventil (21) umgekehrt werden. Dabei saugt Venturi (14) über den Anschlussschlauch (18) das Flüssigkeitsrohstoffgemisch aus dem Rohstoffbehälter (20). Bedingt durch einen von der Drossel (22) erzeugten Staudruck fließt Flüssigkeit von oben über Anschlussschlauch (19) in den Rohstoffbehälter (20). Durch eine Verlängerung des Anschlusses (19) im Behälter (20) kann durch wechselweises Schalten der Ventile (23/24) zusätzlich Luft von oben in den Behälter (20) eingebracht werden, um den Auflösevorgang des Rohstoffes zu beschleunigen.

Zum Entleeren saugt das Venturi (14) bei geöffnetem Ventil (24) über den Ansaugschlauch (18) die aufgelöste Mischung aus dem Behälter (20), bis die optionale Leererkennung (16) den Vorgang beendet. In der Regel erfolgt das Absaugen des Flüssigkeitsrohstoffgemisches zeitgesteuert. Die Schläuche (18/19) können danach an der Parkstation (17) zurück konnektiert werden.

Zur Drucküberwachung des Rohstoffbehälters (20) dient der Drucksensor (9), der auch an anderer, hier nicht dargestellter Stelle des Hydraulikkreislaufes angebracht werden kann. Dabei kann der Drucksensor (9) die Dichtheit der angeschlossenen Komponenten wie z.B. Transferfilter (11), Transferventil (10) mit überprüfen.

Die Fertiglösung wird aus dem Fertiglösungstank (33) mittels der Pumpe (7) und des Transferventils (10) und des Transferfilters (11) zum Anwendungsort oder in bereitgestellte Tanks gepumpt.

Die Spülung der Mischanlage (61) erfolgt im Wechsel so, dass alle Leitungen (26/63) durchflossen werden, wobei der Reinigungssprühkopf (27) so angebracht ist, dass die gesamte Innenoberfläche des Fertiglösungsbehälters (33) besprüht werden kann.

Zur Verbesserung der Reinigungseffektivität kann optional eine Reinigungs-/Desinfektionsvorrichtung (25) in die Leitung (26) zum Fertigungslösungsbehälter (33) eingebaut werden. Zur Überprüfung der Konzentration sowohl der fertigen Mischung als auch einer Desinfektionslösung kann die Leitfähigkeitsmessung (13) eingesetzt werden.

Figur 2 zeigt eine vereinfachte Zudosiereinrichtung, die in der Regel aus einem Eingangsventil (2), einem Flussmesser oder Wasserzähler (4) und den Niveau Sensoren (31/29) besteht. Dabei kann im einfachsten Falle auch ohne Flussmesser (4) dosiert werden, weil die Genauigkeit des zugeführten Volumens sowohl durch den Niveau Sensor (29) als auch als Füllstandshöhe mittels eines transluzenten Bereichs und oder einer umlaufenden Tankmarkierung durch den Anwender kontrollierbar ist. Damit kann auch eine eventuelle volumenrelevante fehlerhafte Schräglage des Tanks erkannt werden.

Optional wird die Software des Rechners (60) so ausgestattet, dass ein fehlerhaftes offenes Ventil (6) mittels Niveausensor (31) überwacht wird und undichte fehlerhafte Ventile bzw. Verbindungen (10; 11; 12) mittels Druckhaltetest durch den Drucksensor (9) erfasst werden.

Zum Ausgleich einer Tank-Schräglage kann eine hier nicht dargestellte Lösung wie z.B. nivellierbarer Sockel oder eine vergleichbare Lösung genutzt werden. Um alters- oder lastbedingte volumenrelevante Tankausbauchungen zu vermeiden, kann der Tank (33) mittig einen formstabilen Tank-Gurt erhalten. Die oben aufgeführte Zudosierung ist nur für fixe Zulaufniveaus geeignet. Die Erfindung sieht vor, auch mehrere umlaufende Niveau-Überwachungsmarkierungen (41) und für jedes gewünschtes zu dosierendes Volumen einen Niveau Sensor (29) anzubringen.

Das Behältnis (42) ist ein wiederbefüllbarer Rohrstoffbehälter mit geradem, schrägem oder konischem oder eingebauchten Boden und einem unteren tangentialen Zulauf (34), der von vorne gesehen unten rechts mit einem großen Durchmesser z.B. 32 mm angeordnet ist.

Der Behälter weist einen oberen tangentialen Ablauf (35), der von vorne gesehen links oben angeordnet ist, und einen Durchmesser von z. B. 15 mm hat und einen weiteren unteren tangentialen Ablauf (52) auf, der von vorne gesehen unten links angeordnet ist und einen Durchmesser von z. B. 20 mm aufweist. Das Behältnis (42) hat einen verschließbaren Deckel (37) der in einer trichterförmigen Einbuchtung angeordnet ist, um zum einen den Freiraum des Rotationsparaboloiden während des Mischvorganges im Behälter auszufüllen und zum anderen die Zuführung des Salzes ohne Verschütten zu ermöglichen.

Der untere optionale Abfluss (52) dient dem zusätzlichen Austrag des eingefüllten Rohstoffes bei geringen Durchflüssen durch den Behälter (42) zu Beginn des Mischvorganges, z.B. bei Verklumpung, und wird an der Außenwand des Behälters (42) so angeordnet, dass er am oberen Ende in den Ablauf (35) einmündet. Zur Vereinfachung der Salzzuführung bei geöffneten Deckel (37) erhält der Behälter eine obere Beutelablage (62) die als Plattform ausgebildet ist. An den Rechner (60) der Mischanlage (61) kann ein Barcodeleser zur Verifizierung des Rohstoffes angeschlossen werden.

Figur 2 zeigt außerdem eine chemische Dosier-/Reinigungsvorrichtung (25). Bei geöffnetem Ventil (63) und geschlossenen Ventilen (24/40/23) kann mittels Venturi (14) Desinfektions-/Reinigungsmittel aus dem Kanister (38) angesaugt werden, bis der Leitfähigkeits-Temperatur-Sensor (13) die gewünschte Zielleitfähigkeit registriert. Die Niveauüberwachungskammer (39) kann sowohl als Leererkennung für den Kanister (38) als auch als Schutzsystem für ungewünschtes Ansaugen fungieren. Da das Ventil (40) im Ruhezustand offen ist, muss die niveauüberwachte Kammer (39) entleert sein.

Mit der Vorrichtung (25) ist auch die sichere Zudosierung kritischer flüssiger Substanzen wie z.B. Essigsäure für die Herstellung von HD Konzentraten möglich, falls dieser Rohstoff separat einzubringen wäre.

Anstelle einer chemischen Reinigungsvorrichtung (25) wäre auch eine die Desinfektion betreffende, gleichwertige physikalische Reinigungsvorrichtung (25) mit einer Ozonzelle möglich.

Ebenso ist eine thermische Desinfektion oder eine Kombination thermisch und chemisch mittels der Heizung (8) möglich.

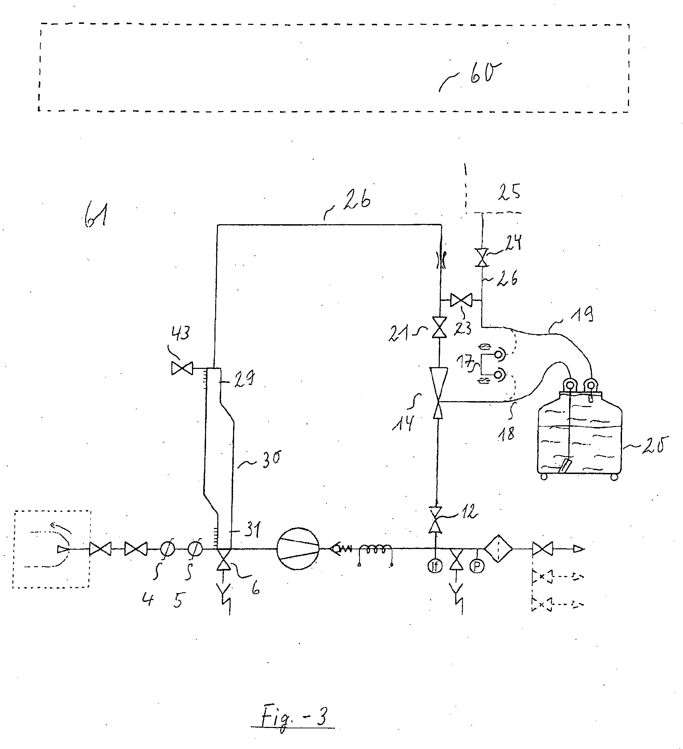

Figur 3 zeigt eine Mischvorrichtung ohne Ansatztank. Dabei werden die Flussmesser (4/5) über die Messkammer (30) verifiziert. Die Füllung des Rohstoffbehälters (20) erfolgt über die Leitung (26), das Ventil (21) über das Venturi (14), den Schlauch (18) oder wechselweise das Ventil (23) und den Schlauch (19) in den Rohstoffbehälter (20), der nur teilweise mit Rohstoff (21) gefüllt ist, um die zusätzliche zur Verdünnung benötigte Flüssigkeitsmenge aufnehmen zu können.

Der Mischvorgang erfolgt wie bereits in Figur 1 beschrieben. Zur Verbesserung der Mischeffizienz kann dabei ein statischer Mischer (66) wie in Fig. 2 dargestellt mit eingesetzt werden. Vorteilhafterweise ist hierbei der Rohstoffbehälter (20) mobil und kann nach durchgeführtem Mischvorgang als Fertiglösungsbehälter zum Einsatzort gebracht werden. Ebenso ist ein Transfer wie in Figur 1 beschrieben möglich.

Bei stationären Rohstofftanks (20) besteht die Möglichkeit, die Mischanlage (61) mobil zu gestalten.

Die Messkammer ist in drei Abschnitte mit einem mittleren großvolumigeren Teil und zwei Strecken mit kleineren Querschnitten gegliedert. Die beiden Endabschnitte dienen dabei der feineren Auflösung, und dort kann der Füllstand entweder als transparente Messstrecke optisch oder auch kapazitiv mittels Niveausensoren (29/31) bzw. mit anderer Sensortechnik durch den Rechner (60) der Mischanlage (61) ermittelt werden. Der oder auch die von den Flussmessern abgegebenen Werte werden mit dem als bekannt vorausgesetzten Volumen der Messkammer (30) verglichen bzw. verifiziert. Dabei kann die Messkammer (30) auch konstruktiv andere geometrische Formen aufweisen. Das Ventil (43) dient der Belüftung der Messkammer (30), um diese beispielsweise vor Beginn einer Messung über das Abflussventil (6) zu entleeren.

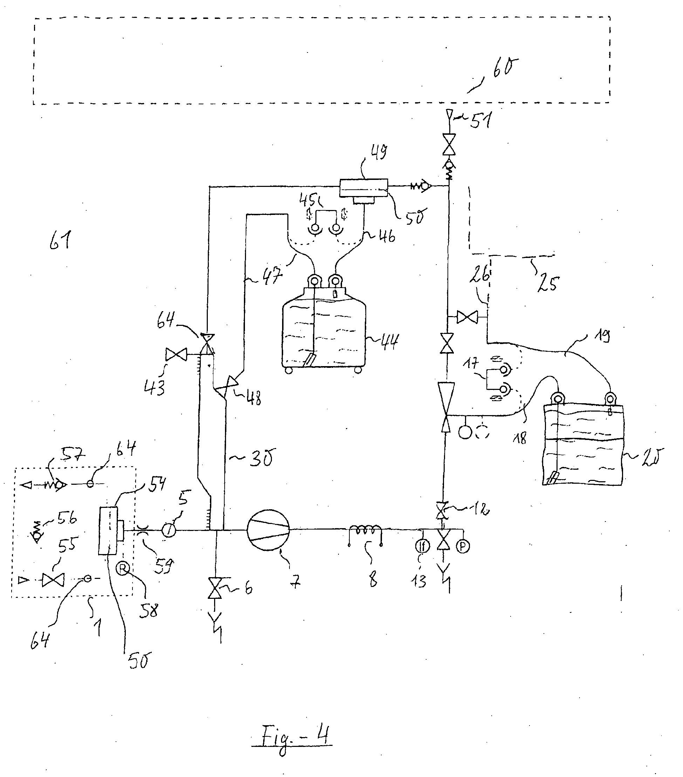

Figur 4 zeigt den Einsatz einer Mischvorrichtung mit einem sterilen Flüssigkeitsversorgungsanschluss (1), wobei die Flüssigkeit einer vorgeschalteten hier nur teilweise dargestellten Umkehrosmose (RO) über den Eingangsfilter (54) zur Messkammer (30) eingebracht wird.

Dabei dienen die Flussmesser (64), die als integraler Bestandteil einer Umkehrosmose-Anlage symbolisch dargestellt sind, als Betriebsflussmesser und der Flussmesser (5) als Schutzsystemflussmesser. Das Ventil (55), ebenfalls integraler Bestandteil einer Umkehrosmoseanlage, dient als Schutzventil für fehlerhafte Zudosierung, wobei in diesem Fall auch die Umkehrosmose-Anlage abgeschaltet werden kann. Die Lage der dargestellten Flusssensoren ist innerhalb der RO auch an anderen dem funktionellen Zweck dienenden Stellen möglich. Anstelle einer RO ist auch der Einsatz einer in einem Tank bereitgestellten Flüssigkeit mit Druckerhöhungsanlage möglich.

Zur Validierung des Eingangsfilters (54) kann mittels Luftanschluss (51) Druckluft auf die Sekundärseite des Eingangsfilters gefördert und die Flüssigkeit aus dem Sekundärraum verdrängt werden. Bei einer intakten Eingangsfiltermembran (50) kann kein Druckabfall durch den Drucksensor (58) registriert werden, weil die intakte Membrane (50) eine luftundurchlässige Funktion hat.

Figur 4 zeigt ebenfalls einen zusätzlichen Füllfilter (49), der auch als Sterilfilter ausgebildet werden kann. Die zugeführte Flüssigkeit wird über die Filtermembran (50) und den Füllanschluss (46) flüssigkeitsberuhigt in den Füllbehälter (44) eingebracht. Die Zirkulation erfolgt über die Leitung (47), das Ventil (48) und die Pumpe (7).

Mittels der optionalen Heizung (8) und der Messeinrichtung (13) kann die Fertiglösung auf eine gewünschte Temperatur und Konzentration eingestellt werden. Dabei bietet der Rechner (60) der Mischanlage (61) auch die Möglichkeit einer Registrierung der verwendeten Rohstoffe bzw. des erzeugten Produkts beispielsweise mittels Barcodeleser und angeschlossenem Drucker. Die Leitungen (18,19/46,47) können dabei aus Gründen der einfachen Handhabung jeweils zu einem Zwillingsschlauch mit jeweils einer Kupplung zusammengefasst werden. Nach Abschluss der Mischung und anschließender Leerung des Rohstoffbehälters (20) wird dieser entsorgt. Die Schläuche (18,19) werden auf die Parkstation (17) und die Schläuche bzw. Kupplungen (46,47) auf die Parkstation (45) zurückgesteckt. Die Mischanlage (61) ist danach wieder betriebsbereit und kann gespült bzw. desinfiziert werden. Der Fertiglösungsbehälter (44) kann zum Einsatzort gebracht und dort vorzugsweise mittels Luftdruck an der Kupplungsstelle (46) und einem Einmalartikel mit Filter an der Kupplungsstelle (47) patientennah verarbeitet werden. Die Filter (49/54) sind vorteilhafterweise als Sterilfilter ausgebildet; dabei kann wie bereits beschrieben die Membranvalidierung mittels Luftdruck erfolgen.

Es wird betont, dass die Erfindung nicht auf die beschriebenen und dargestellten Ausführungsformen beschränkt ist. Vielmehr sind alle offenbarten Merkmale der Ausführungsformen auch einzeln untereinander anders als oben beschrieben miteinander kombinierbar.

**Legende**

| | |
|---|---|
| 1. | Flüssigkeitsversorgungsanschluss |
| 2. | Betriebsventil |
| 3. | Schutzventil |
| 4. | Betriebsflussmesser |
| 5. | Schutzsystemflussmesser |
| 6. | Abflussventil |
| 7. | Zirkulationspumpe |
| 8. | Optionale Heizung |
| 9. | Drucksensor |
| 10. | Transferventil |
| 11. | Transferfilter |
| 12. | Füllhilfsventil |
| 13. | Temperatur / Leitfähigkeitsmessung |
| 14. | Mischventuri |
| 15. | Saugleitung |
| 16. | Optionale Leererkennung |
| 17. | Parkposition Kupplungen Wechselbehälter |
| 18. | Anschlussschlauch Sekundärmischung |
| 19. | Anschlussschlauch Sekundärmischung |
| 20. | Rohstoffbehälter |
| 21. | Mischventil |
| 22. | Drossel |
| 23. | Sekundärmischventil |
| 24. | Spülventil |
| 25. | Desinfektionseinrichtung (optional) |
| 26. | Spülleitung/ Belüftung |
| 27. | Reinigungssprühkopf |
| 28. | Entlüftungsfilter |
| 29. | Niveausensor |
| 30. | Messkammer (optional) |
| 31. | Niveausensor |
| 32. | Überlaufleckage |
| 33. | Fertiglösungsbehälter (Ansatzbehälter mit Restentleerung) |
| 34. | Unterer tangentialer Einlauf Rohstoffbehälter |
| 35. | Ober tang. Ablauf Rohstoffbehälter |
| 36. | Optionale Beipassleitung |
| 37. | Befülldeckel |
| 38. | Desinfektionsmittelkanister/ Säurekanister |
| 39. | Niveau-Überwachungskammer |
| 40. | Belüftungsventil |
| 41. | Niveau-Überwachungsmarkierung |
| 42. | Vor Ort Wiederbefüllbarer Wechselbehälter |
| 43. | Belüftungsventil |
| 44. | Mobiler Fertiglösungsbehälter |
| 45. | Parkposition Kupplungen Fertiglösungsbehälter |
| 46. | Füllanschluss |
| 47. | Entleeranschluss |
| 48. | Absaugventil |
| 49. | Füllfilter |
| 50. | Filtermembrane |
| 51. | Druckluftanschluss |
| 52. | Unterer tangentialer Abfluss |
| 53. | Rotationsparabolid |
| 54. | Eingangsfilter |
| 55. | Reinstwassersperrventil |
| 56. | Überströmventil |
| 57. | Sperrventil |
| 58. | Drucksensor |
| 59. | Druckregler oder auch Konstantflussventil |
| 60. | Rechner der Mischanlage |
| 61. | Mischanlage |
| 62. | Beutelablage |
| 63. | Hauptmischleitung |
| 63.a | Tankauslauf/Füllleitung |
| 63.b | Ventil |
| 64. | Füllventil |
| 65. | Konvergenzkammer |
| 66. | Divergenzkammer |

## Patentansprüche

1. Mischvorrichtung zur Herstellung gebrauchsfertiger medizinischer Spüllösungen, insbesondere für Hämodialysekonzentrate, mit einer Reinstwasserquelle (1), die über eine Zulaufleitung mit einem Rezirkulationskreislauf verbunden ist, in den eine Pumpe (7) eingeschaltet ist, mit einem Rechner (60) und
mit einer Sekundärmischung-Anschlussleitung (18), die mit einem Rohstoffbehälter (20) verbindbar ist, der vor Beginn des Mischvorgangs pulverige und/oder granulierte und/oder geschlämmte Rohstoffe enthält, die mit dem Reinstwasser gemischt werden sollen,
wobei in den Rezirkulationskreislauf in Strömungsrichtung nacheinander ein Mischventurirohr (14) mit einer Konvergenzkammer (65) und einer
Divergenzkammer (66) und ein Mischventil (21) eingeschaltet sind, und wobei die Sekundärmischung-Anschlussleitung (18) mit der Ansaugstelle des Venturirohrs (14) verbunden ist,
**dadurch gekennzeichnet dass** eine weitere Sekundärmischung-Anschlussleitung (19) mit dem Rohstoffbehälter (20) verbindbar ist und nach einem Sekundärmischventil (23) in den Rezirkulationskreislauf einmündet.

2. Mischvorrichtung nach Anspruch 1,
**dadurch gekennzeichnet,**
**dass** von der weiteren Sekundärmischung-Anschlussleitung (19) zwischen dem Rohstoffbehälter (20) und dem Sekundärmischventil (23) eine Spül-/Belüftungsleitung (26) abzweigt, in die ein Spülventil (24) eingeschaltet ist.

3. Mischvorrichtung nach Anspruch 1 oder 2,
**dadurch gekennzeichnet,**
**dass** stromabwärts der Einmündung der weiteren Sekundärmischung-Anschlussleitung (19) in die Rezirkulationsleitung (63) eine Drossel (22) in die Hauptmischleitung (63) eingeschaltet ist.

4. Mischvorrichtung nach den Ansprüchen 1 bis 3,
**dadurch gekennzeichnet,**
**dass** in den Rezirkulationskreislauf ein Fertiglösungsbehälter (33) eingeschaltet ist, wobei eine Hauptmischleitung (63) des Rezirkulationskreislaufs oben in den Fertiglösungsbehälter (33) einmündet.

5. Mischvorrichtung nach den Ansprüchen 1 bis 4,
**dadurch gekennzeichnet,**
**dass** die Spül-/Belüftungsleitung (26) oben in den Fertiglösungsbehälter (33) einmündet.

6. Mischvorrichtung nach den Ansprüchen 1 bis 5,
**dadurch gekennzeichnet,**
**dass** der Rohstoffbehälter (42) mit Rohstoffen wiederbefüllbar ist.

7. Mischvorrichtung nach den Ansprüchen 1 bis 3,
**dadurch gekennzeichnet,**
**dass** der Rohstoffbehälter (20) als Fertiglösungsbehälter dient (Figur 3).

8. Mischvorrichtung nach den Ansprüchen 1 bis 7,
**dadurch gekennzeichnet,**
**dass** in die Zulaufleitung oder den Rezirkulationskreislauf wenigstens ein mit dem Rechner (60) verbundener Flussmesser (4,5) eingeschaltet ist und dass stromabwärts davon eine volumetrische Messkammer (30) angeordnet ist, in der der Füllstand der Messkammer (30) ermittelt wird, wobei der ermittelte Wert durch den Rechner (60) mit dem Messwert des wenigstens einen Flussmessers (4,5) verglichen und dessen Messwert erforderlichenfalls korrigiert wird.

9. Mischanlage nach Anspruch 8,
**dadurch gekennzeichnet,**
**dass** zwei Flussmesser (4,5) hintereinander angeordnet sind, die als Betriebsflussmesser (4) und Schutzsystemflussmesser (5) redundant arbeiten und von dem Rechner (60) der Mischvorrichtung überwacht werden.

10. Mischvorrichtung nach den Ansprüchen 1 bis 9,
**dadurch gekennzeichnet,**
**dass** von dem Rezirkulationskreislauf eine Transferleitung mit wenigstens einem Transferventil (10) abzweigt.

## Claims

1. A mixing device for producing ready-to-use medical irrigation solutions, particularly for haemodialysis concentrates, with a high purity water source (1), which is connected via a feed line to a recirculation circuit, connected into which is a pump (7), with a computer (60) and with a secondary mixture connecting line (18), which is connectable to a raw material container (20), which contains raw materials in pulverulent and/or granulated and/or slurry form, which are to be mixed with the high purity water, wherein connected into the recirculation circuit sequentially in the flow direction there are a mixing venturi pipe (14) with a convergence chamber (65) and a divergence chamber (66) and a mixing valve (21) and wherein the secondary mixture connecting line (18) is connected to the inlet area of the venturi pipe (14), **characterised in that** a further secondary mixture connecting line (19) is connectable to the raw material container (20) and communicates after a secondary mixing valve (23) with the recirculation circuit.

2. A mixing device as claimed in Claim 1, **characterised in that** branching off from the further secondary mixture connecting line (19) between the raw material container (20) and the secondary mixing valve (23) there is a flushing/aeration line (26), connected into which there is a flushing valve (24).

3. A mixing device as claimed in Claim 1 or 2, **characterised in that** connected into the main mixture line (63) downstream of the communication of the further secondary mixture connecting line (19) with the recirculation line (63) there is a throttle (22).

4. A mixing device as claimed in Claims 1 to 3, **characterised in that** connected into the recirculation circuit there is a final solution container (33), wherein a main mixture line (63) of the recirculation circuit communicates with the top of the final solution container (33).

5. A mixing device as claimed in Claims 1 to 4, **characterised in that** the flushing/aeration line (26) communicates with the top of the final solution container (33).

6. A mixing device as claimed in Claims 1 to 5, **characterised in that** the raw material container (42) is refillable with raw materials.

7. A mixing device as claimed in Claims 1 to 3, **characterised in that** the raw material container (20) serves as the final solution container (Figure 3).

8. A mixing device as claimed in Claims 1 to 7, **characterised in that** connected into the feed line or the recirculation circuit there is at least one flow meter (4, 5) connected to the computer (60) and that arranged downstream of it there is a volumetric measuring chamber (30), in which the liquid level of the measuring chamber (30) is determined, wherein the determined value is compared by the computer (60) with the measured value of the at least one flow meter (4, 5) and its measured value is corrected, if necessary.

9. A mixing device as claimed in Claim 8, **characterised in that** two flow meters (4, 5) are arranged in series, which operate redundantly as an operational flow meter (4) and a protective system flow meter (6) and are monitored by the computer (60) of the mixing device.

10. A mixing as claimed in Claims 1 to 9, **characterised in that** branching off from the recirculation circuit there is a transfer line with at least one transfer valve (10).

## Revendications

1. Dispositif de mélange pour la production de solutions de rinçage médicales prêtes à l'emploi, en particulier pour des concentrés d'hémodialyse, comportant une source d'eau pure (1) qui est reliée par une conduite d'alimentation à un circuit de recyclage, dans lequel une pompe (7) est en fonctionnement,
et un compteur (60) et
une conduite de raccordement du mélange secondaire (18) qui peut être reliée à un conteneur de matière première (20) qui contient, avant le début du processus de mélange, des matières premières pulvérulentes et/ou granulées et/ou en suspension, qui doivent être mélangées à l'eau pure,
dans lequel, dans le circuit de recyclage, dans le sens du courant, sont en fonctionnement consécutivement un tube de mélange Venturi (14) doté d'une chambre de convergence (65) et d'une chambre de divergence (66) et une vanne de mélange (21), et
dans lequel la conduite de raccordement du mélange secondaire (18) est reliée au point d'aspiration du tube Venturi (14),
**caractérisé en ce que** :
une autre conduite de raccordement de mélange secondaire (19) peut être reliée au conteneur de matière première (20) et débouche après une vanne de mélange secondaire (23) dans le circuit de recyclage.

2. Dispositif de mélange selon la revendication 1,
**caractérisé en ce que** :
partant de l'autre conduite de raccordement du mélange secondaire (19), entre le conteneur de matière première (20) et la soupape de mélange secondaire (23), se divise une conduite de rinçage/aération (26) dans laquelle une vanne de rinçage (24) est en fonctionnement.

3. Dispositif de mélange selon la revendication 1 ou 2,
**caractérisé en ce que** :
en aval de l'embouchure de l'autre conduite de raccordement du mélange secondaire (19) dans la conduite de recyclage (63), un étrangleur (22) est en fonctionnement dans la conduite de mélange principal (63).

4. Dispositif de mélange selon les revendications 1 à 3,
**caractérisé en ce que** :
dans le circuit de recyclage, un conteneur de solution terminée (33) est en fonctionnement, dans lequel une conduite de mélange principal (63) du circuit de recyclage débouche en haut du conteneur de solution terminée (33).

5. Dispositif de mélange selon les revendications 1 à 4,
**caractérisé en ce que** :
la conduite de rinçage/aération (26) débouche en haut du conteneur de solution terminée (33).

6. Dispositif de mélange selon les revendications 1 à 5,
**caractérisé en ce que** :
le conteneur de matière première (42) peut être rempli à nouveau de matières premières.

7. Dispositif de mélange selon les revendications 1 à 3,
**caractérisé en ce que** :
le conteneur de matières premières (20) sert de conteneur de solution terminée (figure 3).

8. Dispositif de mélange selon les revendications 1 à 7,
**caractérisé en ce que** :
dans la conduite d'acheminement ou dans le circuit de recyclage, au moins un débitmètre (4, 5) relié au compteur (60) est en fonctionnement, et **en ce qu'**en aval de celui-ci, une chambre de mesure volumétrique (30) est disposée, dans laquelle l'état de remplissage de la chambre de mesure (30) est déterminé, dans lequel la valeur déterminée par le compteur (60) est comparée à la valeur de mesure d'au moins un débitmètre (4, 5) et sa valeur de mesure est corrigée le cas échéant.

9. Dispositif de mélange selon la revendication 8
**caractérisé en ce que** :
deux débitmètres (4, 5) sont disposés l'un derrière l'autre, qui fonctionnent de façon redondante en tant que débitmètres de fonctionnement (4) et débitmètres de système de protection (5) et sont surveillés par le compteur (60) du dispositif de mélange.

10. Dispositif de mélange selon les revendications 1 à 9,
**caractérisé en ce que** :
partant du circuit de recyclage, se divise une conduite de transfert comportant au moins une vanne de transfert (10).
